# FASCICULE DE BREVET EUROPEEN

(11) **EP 1 323 556 B1**
(45) Date de publication et mention de la délivrance du brevet: **06.10.2004**
(21) Numéro de dépôt: 02028408.9
(22) Date de dépôt: 18.12.2002
(51) Int. Cl.: B60H 3/00

(54) **Dispositif d'odorisation de l'habitacle d'un véhicule automobile**
Duftdiffusionsmittel für einen Fahrzeuginnenraum
Fragrance-supplying means in a vehicle passenger compartment

(30) Priorité: 20.12.2001 FR 0116571
(43) Date de publication de la demande: 02.07.2003
(73) Titulaire: VALEO CLIMATISATION, 78321 La Verrière (FR)
(72) Inventeur: Poitier, Gérard, 92230 Gennevilliers (FR)

(56) Documents cités:
- DE-A- 19 910 227
- FR-A- 2 231 216
- US-A- 5 314 669
- US-A- 5 527 493
- PATENT ABSTRACTS OF JAPAN vol. 007, no. 159 (M-228), 13 juillet 1983 (1983-07-13) -& JP 58 067512 A (NIPPON DENSO KK), 22 avril 1983 (1983-04-22)

## Description

L'invention se rapporte aux dispositifs d'odorisation de l'habitacle des véhicules automobiles.

Elle concerne plus particulièrement un dispositif d'odorisation du type comprenant au moins une cartouche remplaçable contenant un agent aromatique propre à être traversée par un flux d'air à odoriser pour produire un flux d'air odorisé.

On connaît déjà des dispositifs d'odorisation de ce type. Ainsi, la publication de brevet japonais n° 58-81817 décrit un dispositif d'odorisation comprenant une cassette contenant un agent aromatique situé en dérivation du boîtier du dispositif de chauffage et/ou de climatisation du véhicule.

Ce dispositif présente différents inconvénients du fait notamment qu'il ne permet pas de régler l'intensité de diffusion du parfum et qu'il ne comporte qu'une seule cartouche ou cassette d'agent aromatique.

On connaît aussi, d'après la publication FR 2 774 597, un dispositif de diffusion de substance volatile comprenant plusieurs diffuseurs montés en parallèle et entraînés chacun par un moteur pas à pas. Ce dispositif est complexe à réaliser.

L'invention a notamment pour but de surmonter les inconvénients précités.

Elle vise en particulier à procurer un dispositif d'odorisation peu coûteux à fabriquer et susceptible de diffuser une ou plusieurs substances aromatiques avec une intensité réglable.

L'invention propose à cet effet un dispositif d'odorisation du type défini précédemment, lequel comprend une platine munie d'un siège dans lequel débouchent une fenêtre d'entrée pour le flux d'air à odoriser et une fenêtre de sortie pour le flux d'air odorisé, une enveloppe de forme générale cylindrique portée par la platine et propre à loger au moins une cartouche d'agent aromatique disposée dans la direction axiale, ladite cartouche ayant une entrée et une sortie aménagées dans une même face d'extrémité, ainsi qu'un organe de réglage interposé entre le siège de la platine et la face d'extrémité de la cartouche et propre à être amené sélectivement soit dans une position de stockage dans laquelle l'agent aromatique de la cartouche ne communique ni avec la fenêtre d'entrée, ni avec la fenêtre de sortie, soit dans au moins une position de diffusion dans laquelle un flux d'air à odoriser est apte à pénétrer dans l'entrée de la cartouche, à se charger de l'agent aromatique de cette cartouche pour produire un flux d'air odorisé qui s'échappe par la sortie de la cartouche.

Ainsi, le dispositif d'odorisation est de conception simple et est peu coûteux à fabriquer. De plus, le remplacement de la ou des cartouches s'effectue simplement puisqu'il suffit d'extraire une cartouche dans la direction axiale de l'enveloppe pour la remplacer par une autre cartouche.

On comprendra qu'en plaçant le dispositif d'odorisation dans un endroit choisi de l'habitacle du véhicule, le changement d'une cartouche par une autre peut s'effectuer de façon simple.

Par ailleurs, le dispositif de l'invention peut être complètement découplé de l'installation de chauffage et/ou de climatisation du véhicule. Il suffit en effet de prévoir une prise d'air qui alimente la fenêtre d'entrée de la platine et une sortie d'air qui est reliée à la fenêtre de sortie de la platine et qui débouche dans un emplacement choisi de l'habitacle du véhicule.

Avantageusement, la platine comporte une tubulure d'entrée et une tubulure de sortie qui communiquent respectivement avec la fenêtre d'entrée et la fenêtre de sortie.

Dans une forme de réalisation préférée de l'invention, le siège de la platine est de forme annulaire, tandis que la fenêtre d'entrée et la fenêtre de sortie débouchent dans des régions diamétralement opposées du siège, et que l'organe de réglage est un disque rotatif comportant au moins deux ouvertures aménagées en des régions choisies qui, dans une position de diffusion, communiquent respectivement avec la fenêtre d'entrée et la fenêtre de sortie.

Ainsi, l'organe de réglage permet de placer le dispositif soit dans une position de stockage, soit dans au moins une position de diffusion.

De préférence, le dispositif d'odorisation est propre à loger au moins deux cartouches contenant des agents aromatiques différents. En ce cas, l'organe de réglage permet aussi de choisir celui des agents aromatiques qui est à diffuser dans l'habitacle.

De préférence, le siège de la platine comprend une ouverture centrale pour le passage d'un axe rotatif relié à l'organe de réglage.

Selon encore une autre caractéristique de l'invention, le dispositif comporte un actionneur porté par la platine et propre à entraîner l'organe de réglage en rotation pour l'amener dans une position angulaire choisie. Cet organe de réglage peut être choisi parmi les actionneurs mécaniques, électriques et pneumatiques.

Selon encore une autre caractéristique de l'invention, la cartouche comprend au moins une cloison interne propre à définir un circuit de circulation en U ou en zigzag pour le flux d'air entre l'entrée et la sortie de la cartouche. En effet, comme l'entrée et la sortie de la cartouche sont sur une même face d'extrémité, cela permet de réaliser l'arrivée de l'air à odoriser et la sortie de l'air odorisé en des emplacements voisins sur la platine du dispositif.

Selon encore une autre caractéristique de l'invention, le dispositif comprend des moyens d'identification de la cartouche. Ces moyens d'identification peuvent être portés par un bouchon de la cartouche situé à l'opposé de la face d'extrémité de la cartouche.

Il est envisageable aussi d'utiliser des moyens d'identification sous la forme de moyens de reconnaissance du type puces, contacts électriques, codes à barre, etc.

Il est avantageux aussi que l'enveloppe comporte des moyens de maintien pour immobiliser la cartouche dans l'enveloppe.

Dans une forme de réalisation préférée, l'enveloppe est propre à loger deux cartouches disposées adjacentes dans la direction axiale. En ce cas, l'enveloppe comprend avantageusement une paroi cylindrique munie d'une cloison interne diamétrale propre à définir deux logements de forme générale semi-cylindrique pour loger respectivement deux cartouches de forme générale semi-cylindrique, tandis que l'enveloppe comporte une extrémité ouverte formant accès pour la mise en place ou l'enlèvement des cartouches.

Le terme «cylindre» ou «cylindrique» est à comprendre au sens général, ou mathématique du terme, comme désignant une surface définie par des génératrices parallèles s'appuyant sur une courbe fermée, qui n'est pas nécessairement circulaire.

Selon encore une autre caractéristique de l'invention, le dispositif comprend des moyens de fixation pour le montage en un endroit choisi d'un véhicule automobile.

Sous un autre aspect, l'invention concerne un véhicule automobile équipé d'un dispositif d'odorisation tel que défini précédemment.

Le dispositif est avantageusement fixé en un endroit choisi de la planche de bord, en particulier dans la boîte à gants, ou au niveau de la console du véhicule.

Cela permet de fournir un accès aisé à l'enveloppe du dispositif pour l'échange d'une cartouche.

Dans la description qui suit, faite seulement à titre d'exemple, on se réfère aux dessins annexés, sur lesquels :
- la Figure 1 est une vue partielle en perspective d'une boîte à gants de véhicule automobile équipée d'un dispositif d'odorisation selon une première forme de réalisation de l'invention ;
- la Figure 2 est une vue en perspective éclatée du dispositif d'odorisation de la Figure 1 ;
- la Figure 3 est une vue en perspective d'un dispositif d'odorisation selon une deuxième forme de réalisation de l'invention ;
- la Figure 4 est une vue de côté du dispositif de la Figure 3 ;
- la Figure 5 est une vue d'extrémité du dispositif des Figures 3 et 4 ;
- la Figure 6 est une vue en coupe selon la ligne VI-VI de la Figure 4 ;
- la Figure 7 est une vue en coupe selon la ligne VII-VII de la Figure 4 ;
- la Figure 8 est une vue en coupe selon la ligne VIII-VIII de la Figure 7 ;
- les Figures 9A, 9B et 9C sont des vues en coupe selon la ligne IX-IX de la Figure 6 pour trois positions différentes de l'organe de réglage du dispositif d'odorisation ;
- la Figure 10 est une vue d'extrémité de deux cartouches dans une autre réalisation ;
- les Figures 11A, 11B et 11C sont des vues en coupe d'un autre organe de réglage pour trois positions différentes de celui-ci ;
- les Figures 12A et 12B montrent deux positions d'un organe de réglage dans une autre variante de réalisation ;
- la Figure 13 est une vue en coupe d'une cartouche dans une autre réalisation ;
- la Figure 14 est une vue en coupe du dispositif d'odorisation et de la cartouche de la Figure 13 ;
- les Figures 15 et 16 montrent respectivement en coupe et en perspective deux cartouches dont les bouchons forment des boutons de commande ;
- la Figure 17 est une vue en perspective éclatée analogue à la Figure 2 dans une autre variante de réalisation ;
- la Figure 18 est une vue en coupe d'une cartouche munie d'un moyen d'identification ; et
- la Figure 19 est une vue en perspective de deux cartouches plates superposées.

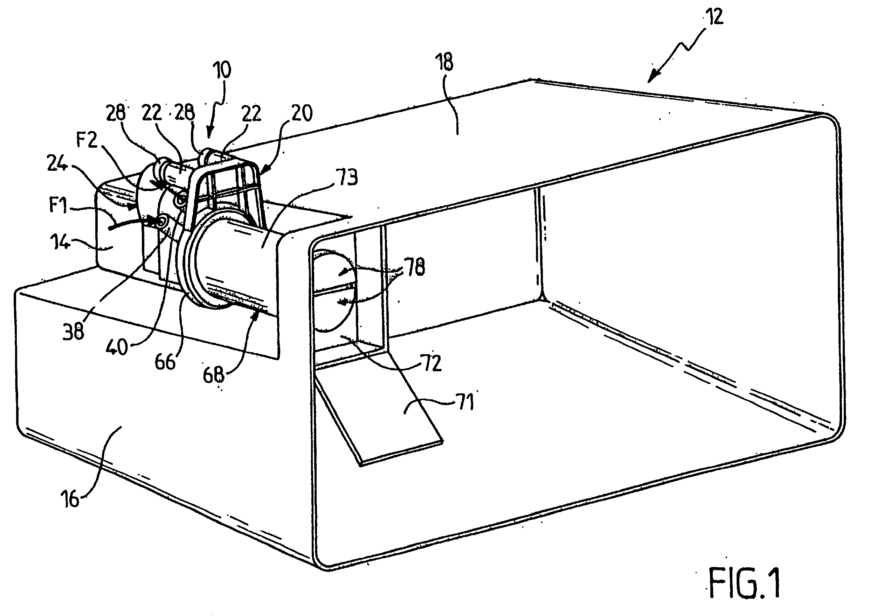

On se réfère d'abord à la Figure 1 qui représente un dispositif d'odorisation 10 selon l'invention, implanté au moins en partie dans une boîte à gants 12 d'un véhicule automobile. Le dispositif d'odorisation est placé dans une encoignure 14 à la jonction d'une paroi latérale 16 et d'une paroi supérieure 18 de la boîte à gants.

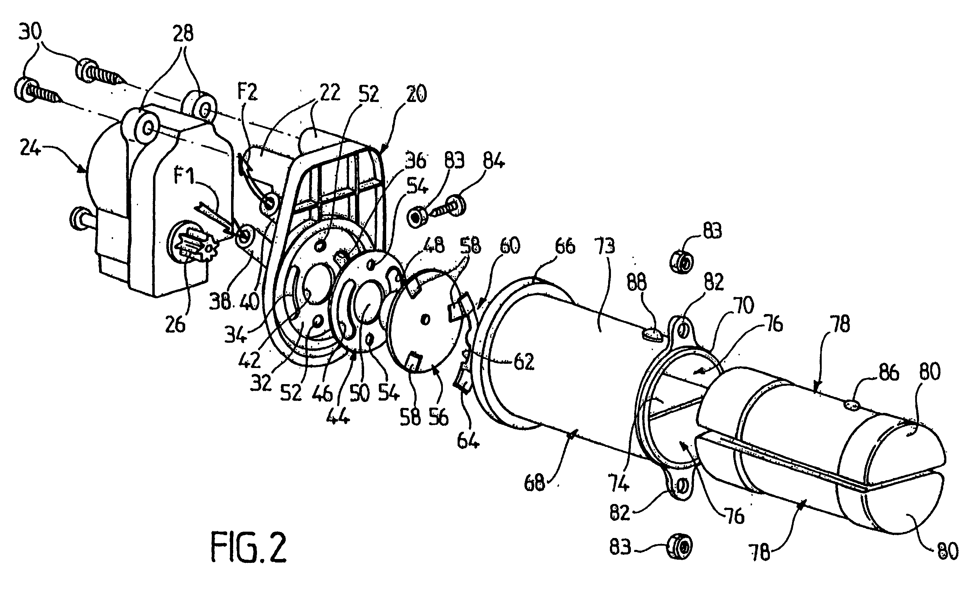

Le dispositif 10 comprend une platine 20 munie d'au moins deux bossages cylindriques 22 (Figures 1 et 2) servant à la fixation d'un actionneur 24 constitué ici par un micromoteur présentant un arbre cannelé 26 d'entraînement. L'actionneur 24 possède au moins deux bossages 28 susceptibles d'être traversés par au moins deux vis 30 venant s'engager dans les bossages 22 de la platine 20 pour assurer la fixation de l'actionneur sur la platine.

La platine 20 est munie d'un siège 32 de forme annulaire qui est dirigé du côté opposé à l'actionneur 24 (Figure 2). Dans le siège 32, débouche une fenêtre d'entrée 34 pour un flux d'air à odoriser et une fenêtre de sortie 36 pour un flux d'air odorisé. Ces deux fenêtres 36 débouchent dans des régions diamétrales opposées et ont chacune la forme d'une ouverture en arc de cercle. Les fenêtres 34 et 36 communiquent respectivement avec une tubulure d'entrée 38 et une tubulure de sortie 40. La tubulure d'entrée 38 est propre à être reliée à une source d'air à odoriser, tandis que la tubulure de sortie 40 est propre à être reliée à une conduite (non représentée) débouchant dans l'habitacle du véhicule, la solution inverse étant également possible.

Le flux d'air à odoriser pénètre dans la tubulure 38, comme montré par la flèche F1 sur les Figures 1 et 2, tandis que le flux d'air odorisé s'échappe par la tubulure 40, comme montré par la flèche F2 sur les Figures 1 et 2

La platine 20 comprend une ouverture centrale 42 de forme circulaire pour le passage de l'arbre cannelé 26. Sur le siège 32 est rapporté un joint d'étanchéité 44 qui présente deux fenêtres 46 et 48 susceptibles de correspondre respectivement avec les fenêtres 34 et 36 du siège, une ouverture centrale 50 propre à correspondre avec l'ouverture centrale 42 de la platine. Le joint est maintenu en position, dans l'exemple, grâce à deux ergots 52 formés en saillie sur le siège de la platine et venant s'engager dans deux trous 54 du joint. D'autres moyens sont envisageables pour assurer le positionnement du joint.

L'arbre 26 est propre à entraîner en rotation un organe de réglage 56 sous la forme d'un disque qui comprend trois ouvertures 58 aménagées en des régions choisies, ici décalées angulairement d'environ 120° les unes des autres. Dans l'exemple, ces ouvertures ont une forme générale carrée. Toutefois, d'autres formes sont possibles. L'organe de réglage 56 reçoit un joint d'étanchéité 60 qui, dans l'exemple, comprend trois branches 62 terminées chacune par une extrémité 64 percée venant s'adapter en regard d'une ouverture de l'organe de réglage.

L'organe de réglage 56 vient coopérer à étanchéité, par l'intermédiaire de son joint 60, avec une première extrémité 66 d'une enveloppe 68 de forme générale cylindrique circulaire, dont l'autre extrémité 70 débouche sur une paroi antérieure 72 située dans l'angle de la boîte à gants 12 (Figure 1). Cette extrémité 70 est accessible lorsque le couvercle (non représenté) de la boîte à gants est en position ouverte. Accessoirement, une trappe 71 spécifique au dispositif d'odorisation 10 peut être prévue dans la boîte à gants, comme montré sur la figure 1.

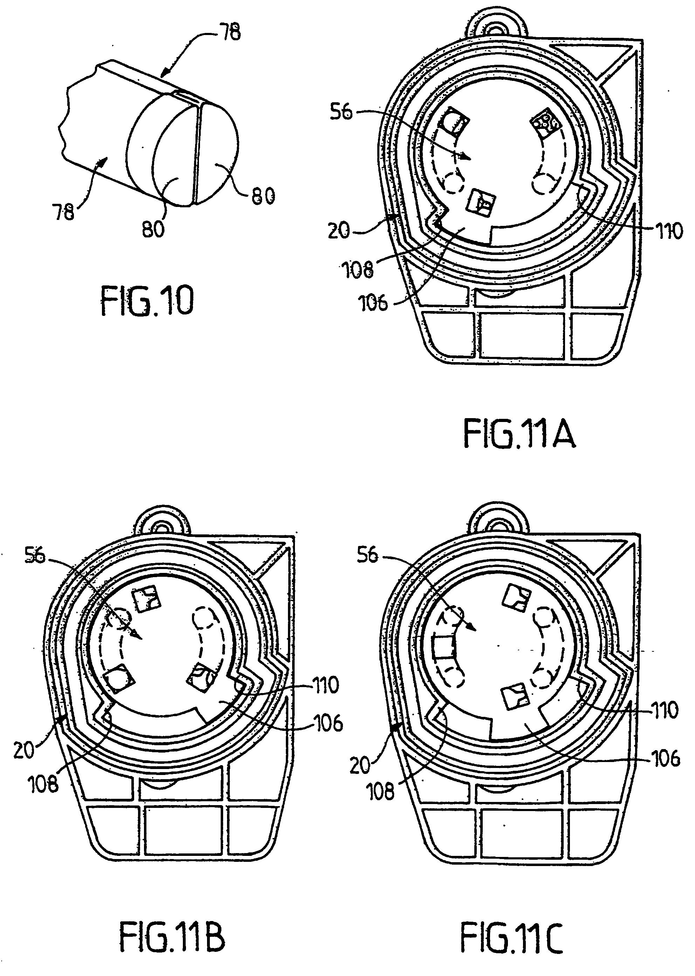

L'enveloppe 68 comprend une paroi cylindrique 73 munie d'une cloison interne diamétrale 74 propre à définir deux logements 76 de forme générale semi-cylindrique destinés à loger respectivement deux cartouches 78 de forme générale semi-cylindrique. Ces cartouches sont destinées à contenir chacune un agent aromatique propre à être traversé par le flux d'air à odoriser pour produire un flux d'air odorisé. Les deux cartouches 78 se terminent chacune par un bouchon d'extrémité 80 qui est visible lorsque le couvercle de la boîte à gants et la trappe 71 sont ouverts, comme on le voit sur la Figure 1. Dans l'exemple représenté, les cartouches sont disposées dans la direction axiale de l'enveloppe en étant juxtaposées verticalement, ce qui permet de définir une cartouche inférieure et une cartouche supérieure. En variante, les cartouches 78 peuvent être juxtaposées horizontalement, comme montré sur la Figure 10, pour procurer une cartouche droite et une cartouche gauche. Les bouchons respectifs 80 des deux cartouches portent des moyens d'identification (non représentés) destinés à permettre de déterminer, par exemple, la nature de l'agent aromatique qu'elle contient.

L'enveloppe 68 comporte deux oreilles 82 qui permettent sa fixation sur la paroi 72 de la boîte à gants. Par ailleurs, la platine est fixée dans l'encoignure 14 par au moins une vis 84, comme montré à la Figure 2. Les points de fixation sont avantageusement découplés de la boîte à gants par des bagues amortisseuses 83, comme montré su la Figure 2.

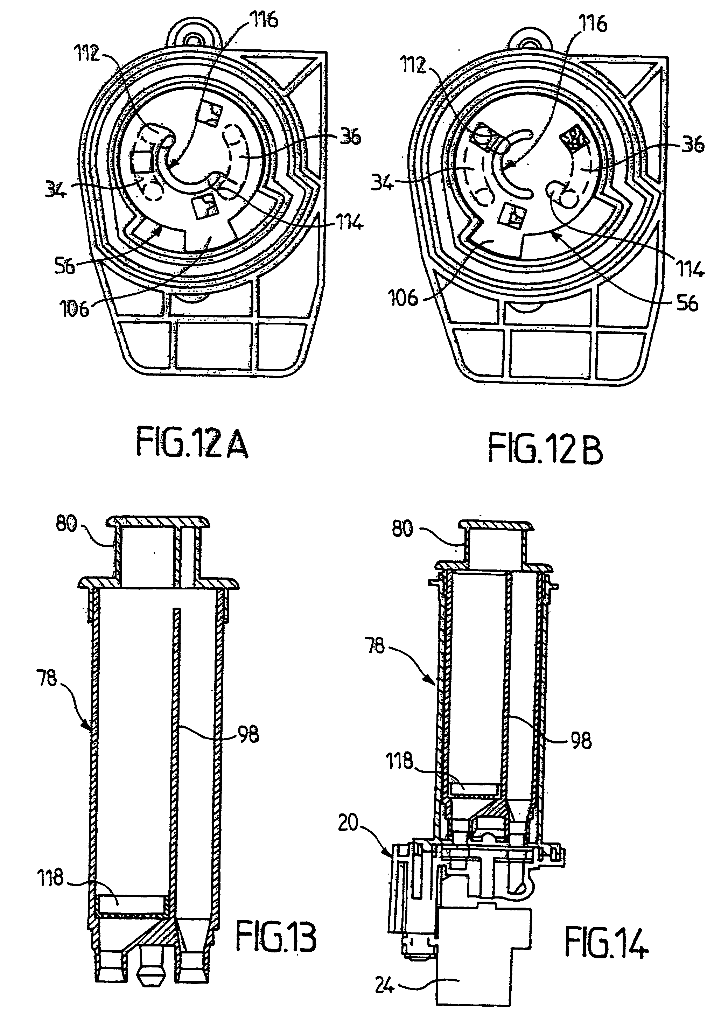

L'actionneur 24 permet d'amener l'organe de réglage 56 dans une multiplicité de positions choisies. Ces positions comprennent notamment une position de stockage dans laquelle l'agent aromatique d'une cartouche ne communique ni avec la fenêtre d'entrée 34, ni avec la fenêtre de sortie 36, si bien qu'aucun air odorisé n'est produit. Les positions comprennent en outre au moins une position de diffusion dans laquelle un flux d'air à odoriser est apte à pénétrer dans une cartouche choisie, par exemple la cartouche supérieure ou la cartouche inférieure, à se charger de l'agent aromatique de la cartouche pour produire un flux d'air odorisé qui s'échappe par la sortie de la cartouche. Des butées peuvent être prévues, comme on le verra plus loin en référence aux Figures 11A, 11B et 11C.

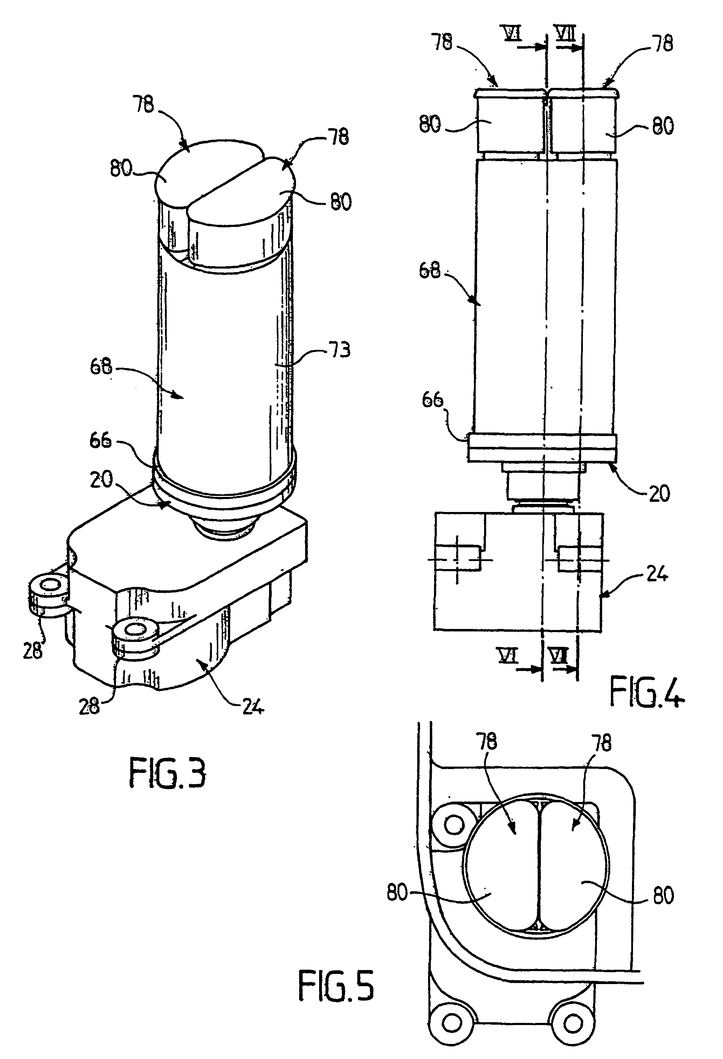

La structure de l'enveloppe et des cartouches sera maintenant décrite plus en détail en référence aux Figures 3 à 9 relatives à une autre forme de réalisation de l'invention. Dans cette seconde forme de réalisation de l'invention, les éléments communs avec la forme de réalisation des Figures 1 et 2 sont désignés par les mêmes références numériques.

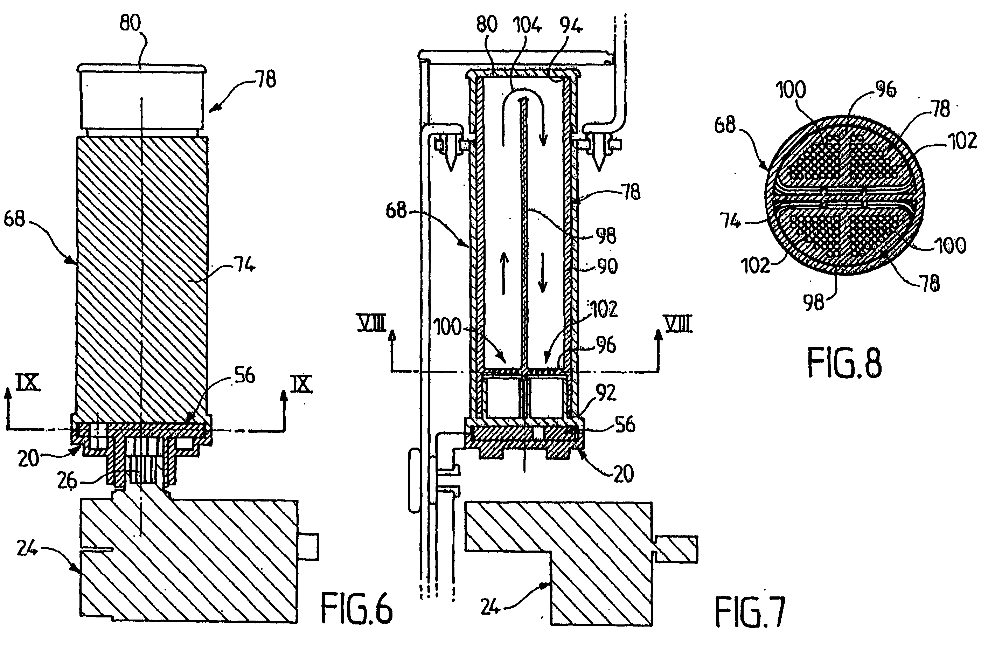

On retrouve sur les Figures 3 et 4 une platine 20 qui a une configuration différente de celle des Figures 1 et 2. Cette platine comprend aussi une tubulure d'entrée et une tubulure de sortie qui ne sont pas représentées pour des raisons de simplification. Sur la platine 20 est fixé aussi un actionneur 24 qui entraîne un arbre cannelé 26 (Figure 6) coopérant avec un organe de réglage 56 (Figures 6 et 7) analogue à celui des Figures 1 et 2.

Comme on peut le voir d'après les Figures 3 à 7, les deux cartouches 78 ont une forme générale semi-cylindrique et viennent se placer dans les logements définis à l'intérieur de l'enveloppe 68. Les deux cartouches 78 sont maintenues immobilisées dans l'enveloppe grâce à des moyens de maintien qui comprennent un ergot 86 issu de la cartouche et venant coopérer avec une ouverture 88 aménagée dans la paroi cylindrique 73 de l'enveloppe (Figure 2). Toutefois, d'autres moyens de maintien sont envisageables. Ainsi, on peut prévoir des moyens de clippage à l'une ou l'autre des extrémités de la cartouche.

En variante, la trappe 71 (Figure 1) peut intégrer des moyens de maintien des cartouches.

Comme on peut le voir sur la Figure 7, chacune des cartouches 78 comprend une paroi latérale 90 de forme cylindrique semi-circulaire qui présente une extrémité ouverte 92 située du côté de l'organe de réglage et une extrémité ouverte 94 destinée à être fermée par le bouchon 80. A l'intérieur de chaque cartouche est prévue une paroi de fond 96 qui est perforée, comme on le voit mieux sur la coupe de la Figure 8. A l'intérieur de la cartouche, s'étend une cloison longitudinale 98 (Figure 7) qui s'étend jusqu'à la paroi de fond 96, mais à distance de l'extrémité 94. Le fond perforé 96 définit une entrée 100 et une sortie 102 pour le flux d'air, ce qui permet de définir un trajet 104 entre l'entrée 100 et la sortie 102 de la cartouche.

Bien entendu, d'autres types de cloisons sont possibles, notamment pour définir un trajet en zigzag. On aperçoit sur la Figure 8 l'entrée 100 et la sortie 102 de la cartouche supérieure, ainsi que l'entrée 100 et la sortie 102 de la cartouche inférieure. Grâce à l'actionneur 24, il est possible d'ouvrir ou de fermer sélectivement l'entrée 100 et/ou la sortie 102 d'une cartouche pour définir différentes positions, comme indiqué précédemment.

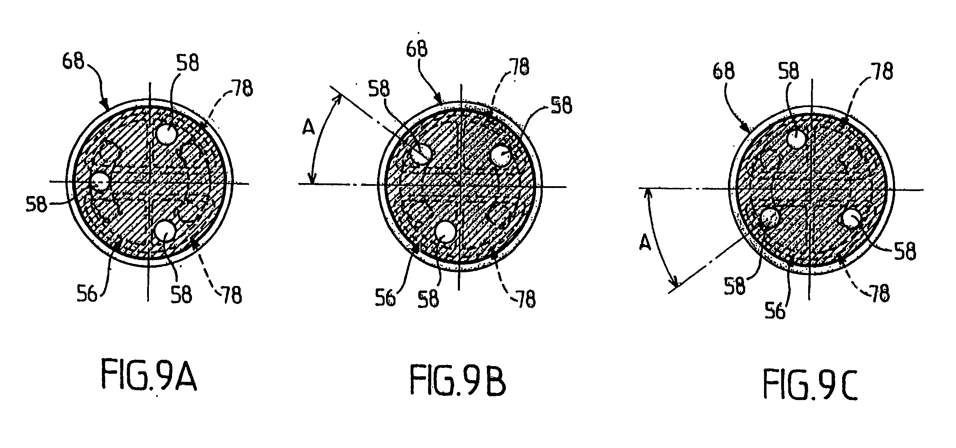

On se réfère maintenant aux Figures 9A, 9B et 9C pour décrire différentes positions d'utilisation.

Dans la position de la Figure 9A, aucune des trois ouvertures 58 ne communique avec l'une ou l'autre des deux cartouches. On se trouve donc dans une position de stockage dans laquelle l'agent aromatique de l'une ou l'autre des cartouches ne communique ni avec la fenêtre d'entrée 34, ni avec la fenêtre de sortie 36.

Dans la position de la Figure 9B, deux des ouvertures 58 (celles situées en partie supérieure de la Figure) communiquent respectivement avec l'entrée et la sortie de la cartouche supérieure. Il en résulte que le flux d'air à odoriser pénètre dans l'entrée de la cartouche supérieure qui se charge de l'agent aromatique de cette cartouche pour produire un flux d'air odorisé qui s'échappe par la sortie de cette cartouche supérieure.

Dans la position de la Figure 9C, deux des ouvertures 58 (celles situées en partie inférieure de la figure) communiquent avec la cartouche inférieure. Il en résulte que le flux d'air à odoriser se charge de l'agent aromatique de la cartouche inférieure.

Le passage de la position de stockage à l'une ou l'autre des positions de diffusion nécessite une rotation d'un angle A dans un sens ou dans l'autre qui, dans l'exemple, est de 37,5°.

Les ouvertures 58 de l'organe de réglage 56 peuvent avoir des formes non circulaires, en particulier des formes progressives, permettant un ajustement du débit d'air qui traverse une cartouche et un réglage d'intensité de l'odorisation. En ce cas, la forme progressive peut se trouver indifféremment sur le joint 64 ou sur l'extrémité 66 du corps 68, selon les contraintes du procédé d'assemblage.

Dans la forme de réalisation représentée, il sera avantageux de prévoir un clapet pour éviter un empoussiérage, dans l'hypothèse où une cartouche serait absente. On comprend que le remplacement d'une cartouche se fait aisément lorsque le couvercle de la boîte à gants est ouvert.

On se réfère maintenant à la Figure 10 qui montre deux cartouches 78 analogues à celles des Figures 1 à 5, mais qui sont juxtaposées horizontalement pour procurer une cartouche droite et une cartouche gauche.

Dans la forme de réalisation des Figures 11A à 11C, l'organe de réglage 56 comprend une saillie radiale 106 propre à coopérer avec deux butées 108 et 110 formées dans la platine 20. Dans la position de la Figure 11A, la saillie 106 vient contre la butée 108 pour assurer une première position de diffusion. Dans la position de la Figure 11B, la saillie 106 vient en butée contre la butée 110 pour assurer une deuxième position de diffusion. La position de la Figure 11C correspond à une position intermédiaire dans laquelle la saillie 106 se trouve entre les butées 108 et 110, pour fournir une position de stockage ou position fermée.

L'organe de réglage 56 représenté aux Figures 12A et 12B s'apparente à celui des Figures 11A à 11C. Il comporte également une saillie 106 pour limiter sa course angulaire.

Dans cette forme de réalisation, les fenêtres 34 et 36 de la platine comportent des aménagements de forme 112 et 114 qui coopèrent avec une rainure 116 aménagée sur l'organe de réglage 56. Dans l'exemple, cette rainure a la forme d'un arc de cercle. On procure ainsi une fonction de dérivation des cartouches dans la position fermée (Figure 12A) ce qui permet la purge du dispositif d'odorisation au moment de l'arrêt. Cela permet de procurer un meilleur confort pour le prochain utilisateur en évitant la pollution d'une fragrance par une autre fragrance. La fonction de purge cesse dès que l'utilisateur demande de l'odorisation et tourne l'organe de réglage vers une position d'utilisation, par exemple celle représentée sur la Figure 12B.

En variante, ce moyen de purge pourrait communiquer avec une autre sortie aboutissant directement avec l'extérieur du véhicule. Un moyen intéressant pour cela serait d'utiliser le conduit de condensat (de l'évaporateur de climatisation) formé en siphon afin de limiter les remontées de parfums.

Dans la forme de réalisation des Figures 13 et 14, la cartouche 78 s'apparente à celle des formes de réalisation précédentes. En particulier, la cartouche comprend une cloison interne 98 pour permettre une circulation du flux d'air en U ou en zigzag.

Pour limiter la perte de charge du flux d'air, on peut prévoir qu'une partie de la cartouche ne contienne pas d'agent aromatique, celui-ci étant généralement présent sous la forme de granulés.

Pour maintenir les granulés, différentes solutions sont possibles. On peut prévoir par exemple une capsule perforée 118. Cette capsule peut être par exemple rapportée ou bien issue de moulage de la cartouche et reliée à cette dernière par une charnière film.

Dans la forme de réalisation des Figures 13 et 14, le bouchon 80 qui ferme la cartouche a une forme étagée pour faciliter la préhension, sans augmenter le volume pris sur la boîte à gants.

Dans une variante de réalisation, on peut prévoir que l'entrée et la sortie d'une cartouche soient échangeables suivant qu'il s'agit d'une cartouche inférieure ou supérieure, ou encore d'une cartouche droite ou gauche. Pour cela, le retour du flux d'air n'est pas assuré par la cartouche, comme dans les formes de réalisation précédentes, mais par un conduit intégré au dispositif.

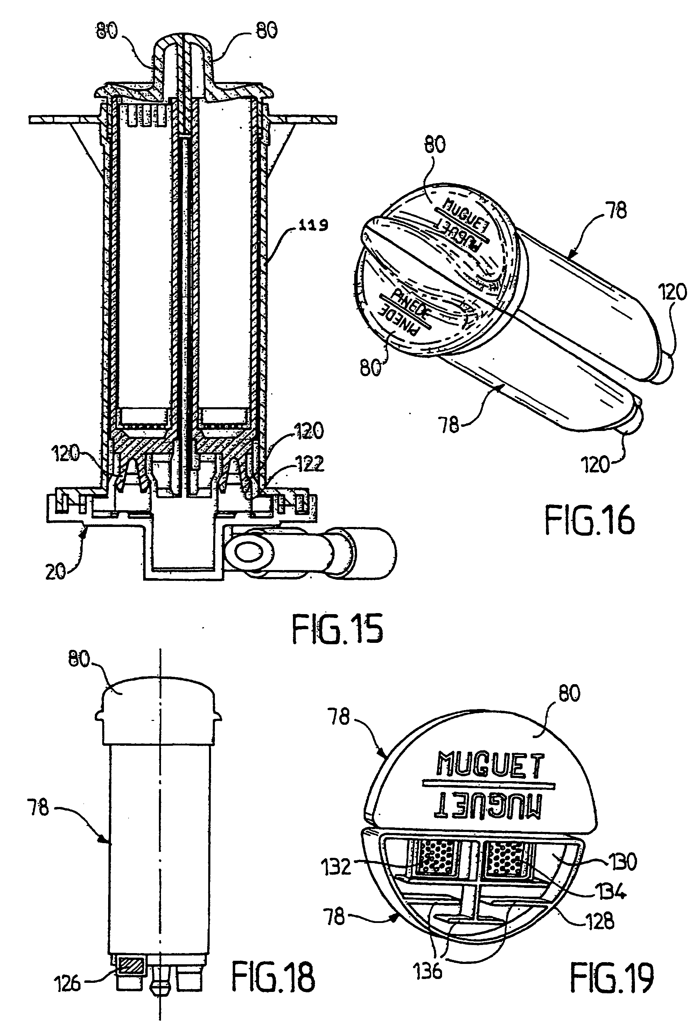

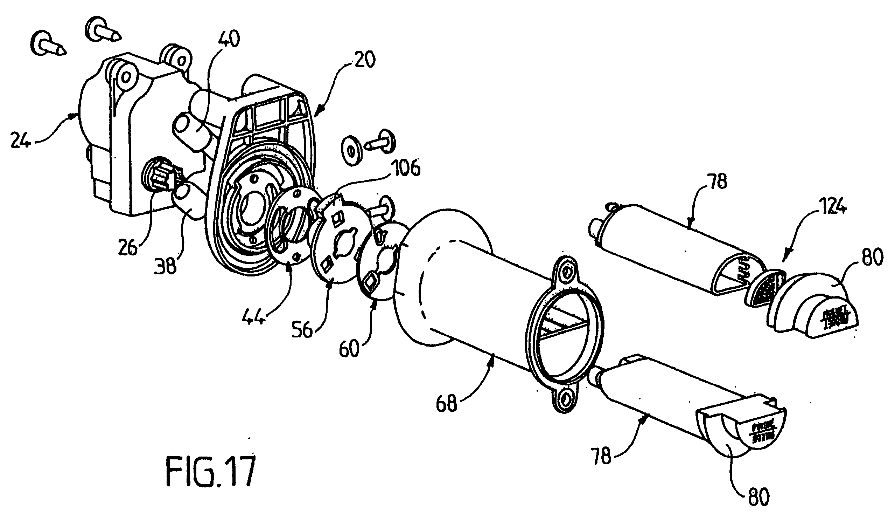

Dans la forme de réalisation des Figures 15 et 16, le dispositif d'odorisation est à commande manuelle. Les bouchons respectifs 80 des deux cartouches 78 sont formés en demi boutons de commande comportant chacun le nom de la fragrance correspondante (Figure 16). Ces boutons 80 permettent de commander la rotation simultanée des deux cartouches 78 dans une enveloppe cylindrique 119, dans laquelle les cartouches sont insérables, comme on le voit sur la Figure 15. A cet effet, chacune des cartouches comprend une extrémité expansible 120 susceptible de s'engager dans une cavité 122, qui va en s'élargissant, de l'organe de réglage.

La Figure 17 illustre un dispositif d'odorisation qui s'apparente étroitement à celui de la Figure 2. On notera que l'organe de réglage 56 comporte une saillie radiale 106 analogue à celle des Figures 11A à 11C et des Figures 12A et 12B.

Chacune des cartouches 78 comprend ici un insert 124 placé dans l'extrémité de la cartouche qui reçoit le bouchon 80. Cet insert permet de procurer une perte de charge qui est spécifique à chaque parfum.

La cartouche représentée à la Figure 18 comprend un organe de positionnement 126 situé du côté de l'extrémité de la cartouche qui est introduit dans l'organe de réglage. Cet organe de positionnement permet l'identification de la cartouche, de sorte qu'une même cartouche puisse être utilisée indifféremment dans deux positions, sans confusion possible.

La Figure 19 montre deux cartouches plates, l'une (en position supérieure) avec son bouchon et l'autre (en position inférieure) sans son bouchon. Chacune des cartouches est délimitée ici par une paroi latérale 128 de forme générale semi circulaire rattachée à une paroi de fond 130 dans laquelle sont ménagées une entrée 132 pour le fluide à odoriser et une sortie 134 pour le fluide odorisé. Ces entrées 132 et 134 sont perforées. A l'intérieur de la cartouche sont prévues des cloisons 136 formant des chicanes et permettant au flux d'air de suivre un parcours en zigzag. On réalise ainsi une cartouche plate d'encombrement réduit par rapport aux cartouches représentées précédemment.

Comme pour les cartouches précédentes, l'agent aromatique est avantageusement présent sous la forme de granulés dans lesquels la fragrance est absorbée.

L'invention n'est pas limitée aux formes de réalisation décrites précédemment mais s'étend à d'autres variantes. En particulier, il est possible de prévoir plus de deux cartouches dans l'enveloppe.

De plus, l'actionneur utilisé pour l'entraînement de l'organe tournant a pu revêtir d'autres formes. Il peut s'agir notamment d'un actionneur mécanique, électrique ou pneumatique.

En particulier, il est envisageable de réaliser l'actionneur sous la forme d'un moto-réducteur avec pilote («driver») et connexions intégrés relié directement à un calculateur par l'intermédiaire d'une liaison filaire ou d'un réseau. Cela permet de constituer un dispositif d'odorisation dont la commande peut s'intégrer ou non dans un tableau de commande du véhicule automobile. La seconde disposition a pour avantage d'avoir un système autonome standard aisément intégrable à tous les véhicules.

Il est possible encore de prévoir d'autres moyens d'identification de la cartouche et notamment des moyens de reconnaissance tels que des puces électroniques, des contacts électriques, des codes à barre, etc.

Dans le cas où le dispositif est prévu pour loger trois cartouches, l'enveloppe définit trois logements ayant une section transversale correspondant à un secteur angulaire d'environ 120° et les cartouches auront une forme homologue. Le dispositif est propre à être alimenté par toute source d'air et en particulier par une dérivation d'un flux d'air qui peut être prélevé notamment sur l'appareil de chauffage et/ou de climatisation du véhicule automobile. La sortie de la platine est reliée à un conduit propre à amener l'air odorisé en tout endroit choisi de l'habitacle du véhicule, notamment au niveau d'une bouche d'aération, dans les buses de dégivrage ou dans l'appareil de chauffage et/ou climatisation lui-même.

Il est bien entendu possible d'implanter le dispositif de l'invention en d'autres endroits de l'habitacle, par exemple dans la planche de bord, au niveau de la console, etc.

## Revendications

1. Dispositif d'odorisation de l'habitacle d'un véhicule automobile, du type comprenant au moins une cartouche remplaçable contenant un agent aromatique propre à être traversée par un flux d'air à odoriser pour produire un flux d'air odorisé,
**caractérisé en ce qu'**il comprend une platine (20) munie d'un siège (32) dans lequel débouchent une fenêtre d'entrée (34) pour le flux d'air à odoriser et une fenêtre de sortie (36) pour le flux d'air odorisé, une enveloppe (68) de forme générale cylindrique portée par la platine (20) et propre à loger au moins une cartouche (78) d'agent aromatique disposée dans la direction axiale, ladite cartouche ayant une entrée (100) et une sortie (102) aménagées dans une même face d'extrémité (96), ainsi qu'un organe de réglage (56) interposé entre le siège (32) de la platine (20) et la face d'extrémité de la cartouche, et propre à être amené sélectivement soit dans une position de stockage dans laquelle l'agent aromatique de la cartouche ne communique ni avec la fenêtre d'entrée (34), ni avec la fenêtre de sortie (36), soit dans au moins une position de diffusion dans laquelle un flux d'air à odoriser est apte à pénétrer dans l'entrée (100) d'une cartouche choisie, à se charger de l'agent aromatique de cette cartouche pour produire un flux d'air odorisé qui s'échappe par la sortie (102) de la cartouche.

2. Dispositif selon la revendication 1, **caractérisé en ce que** la platine (20) comporte une tubulure d'entrée (38) et une tubulure de sortie (40) qui communiquent respectivement avec la fenêtre d'entrée (34) et la fenêtre de sortie (36).

3. Dispositif selon l'une des revendications 1 et 2, **caractérisé en ce que** le siège (32) de la platine (20) est de forme annulaire, **en ce que** la fenêtre d'entrée (34) et la fenêtre de sortie (36) débouchent dans des régions diamétrales opposées, et **en ce que** l'organe de réglage (56) est un disque rotatif comportant au moins deux ouvertures (58) aménagées en des régions choisies qui, dans une position de diffusion, communiquent respectivement avec la fenêtre d'entrée (34) et la fenêtre de sortie (36).

4. Dispositif selon la revendication 3, **caractérisé en ce que** la platine (20) comprend une ouverture centrale (42) pour le passage d'un arbre rotatif (26) relié à l'organe de réglage (56).

5. Dispositif selon l'une des revendications 1 à 4, **caractérisé en ce qu'**il comporte un actionneur (24) porté par la platine (20) et propre à entraîner l'organe de réglage (56) en rotation pour l'amener dans une position angulaire choisie.

6. Dispositif selon la revendication 5, **caractérisé en ce que** l'actionneur (24) est choisi parmi les actionneurs mécaniques, électriques et pneumatiques.

7. Dispositif selon l'une des revendications 1 à 6, **caractérisé en ce que** la cartouche (68) comprend au moins une cloison interne (98) propre à définir un circuit de circulation en U ou en zigzag pour le flux d'air entre l'entrée (100) et la sortie (102) de la cartouche.

8. Dispositif selon l'une des revendications 1 à 7, **caractérisé en ce qu'**il comprend des moyens d'identification de la cartouche.

9. Dispositif selon la revendication 8, **caractérisé en ce que** les moyens d'identification sont portés par un bouchon (80) de la cartouche (78) situé à l'opposé de la face d'extrémité de la cartouche.

10. Dispositif selon la revendication 8, **caractérisé en ce que** les moyens d'identification sont des moyens de reconnaissance du type puces, contacts électriques, codes à barre.

11. Dispositif selon l'une des revendications 1 à 10, **caractérisé en ce que** l'enveloppe (68) comporte des moyens de maintien (86, 88) pour immobiliser la cartouche dans l'enveloppe.

12. Dispositif selon l'une des revendications 1 à 11, **caractérisé en ce que** l'enveloppe (68) est propre à loger deux cartouches (78) disposées adjacentes dans la direction axiale.

13. Dispositif selon la revendication 12, **caractérisé en ce que** l'enveloppe (68) comprend une paroi cylindrique (72) munie d'une cloison interne diamétrale (74) propre à définir deux logements (76) de forme générale semi-cylindrique pour loger respectivement deux cartouches de forme générale semi-cylindrique, et **en ce que** l'enveloppe (68) comporte une extrémité ouverte (70) formant accès pour la mise en place ou l'enlèvement des cartouches.

14. Dispositif selon l'une des revendications 1 à 13, **caractérisé en ce qu'**il comprend des moyens de fixation pour le montage en un endroit choisi d'un véhicule automobile.

15. Véhicule automobile, **caractérisé en ce qu'**il est équipé d'un dispositif d'odorisation selon l'une des revendications 1 à 14.

16. Véhicule automobile selon la revendication 15, **caractérisé en ce que** le dispositif d'odorisation est fixé en un endroit choisi de la planche de bord, en particulier dans la boîte à gants (12), au niveau de la console du véhicule.

## Claims

1. Device for odorizing the passenger compartment of a motor vehicle, of the type comprising at least one replaceable cartridge containing an aromatizing agent suitable for a stream of air to be odorized to pass through it in order to produce a stream of odorized air,
**characterized in that** it comprises a mounting plate (20) provided with a seat (32), opening into which seat are an inlet port (34) for the stream of air to be odorized and an outlet port (36) for this stream of odorized air, a casing (68) of cylindrical general shape supported by the mounting plate (20) and suitable for housing at least one aromatizing-agent cartridge (78) placed in the axial direction, the said cartridge having an inlet (100) and an outlet (102) provided in one and the same end face (96), and also an adjustment member (56) which is interposed between the seat (32) of the mounting plate (20) and the end face of the cartridge, and is suitable for being selectively brought either into a storage position, in which the aromatizing agent of the cartridge communicates neither with the inlet port (34) nor with the outlet port (36), or into at least one diffusion position, in which a stream of air to be odorized is capable of entering the inlet (100) of a chosen cartridge and of being charged with the aromatizing agent of this cartridge in order to produce a stream of odorized air that escapes via the outlet (102) of the cartridge.

2. Device according to Claim 1, **characterized in that** the mounting plate (20) has an inlet nozzle (38) and an outlet nozzle (40) that communicate with the inlet port (34) and the outlet port (36), respectively.

3. Device according to either of Claims 1 and 2, **characterized in that** the seat (32) of the mounting plate (20) is of annular shape, **in that** the inlet port (34) and the outlet port (36) open into diametrically opposed regions and **in that** the adjustment member (56) is a rotary disc having at least two openings (58) provided in chosen regions which, in a diffusion position, communicate with the inlet port (34) and the outlet port (36), respectively.

4. Device according to Claim 3, **characterized in that** the mounting plate (20) includes a central opening (42) for the passage of a rotary shaft (26) connected to the adjustment member (56).

5. Device according to one of Claims 1 to 4, **characterized in that** it includes an actuator (24) that is supported by the mounting plate (20) and designed to rotate the adjustment member (56) in order to bring it into a chosen angular position.

6. Device according to Claim 5, **characterized in that** the actuator (24) is chosen from mechanical, electrical and pneumatic actuators.

7. Device according to one of Claims 1 to 6, **characterized in that** the cartridge (68) includes at least one internal partition (98) suitable for defining a U-shaped or zigzag-shaped flow circuit for the stream of air between the inlet (100) and the outlet (102) of the cartridge.

8. Device according to one of Claims 1 to 7, **characterized in that** it includes cartridge identification means.

9. Device according to Claim 8, **characterized in that** the identification means are carried by a plug (80) of the cartridge (78) that lies on the opposite side from the end face of the cartridge.

10. Device according to Claim 8, **characterized in that** the identification means are recognition means of the chip, electrical contact or bar code type.

11. Device according to one of Claims 1 to 10, **characterized in that** the casing (68) includes retention means (86, 88) for immobilizing the cartridge in the casing.

12. Device according to one of Claims 1 to 11, **characterized in that** the casing (68) is designed to house two cartridges (78) that are placed so as to be adjacent along the axial direction.

13. Device according to Claim 12, **characterized in that** the casing (68) comprises a cylindrical wall (72) provided with a diametral internal partition (74) suitable for defining two housings (76) of semicylindrical general shape in order to house two respective cartridges of semicylindrical general shape and **in that** the casing (68) has an open end (70) that forms an access for fitting or removing the cartridges.

14. Device according to one of Claims 1 to 13, **characterized in that** it includes fastening means for mounting it at a chosen place in a motor vehicle.

15. Motor vehicle, **characterized in that** it is equipped with an odorizing device according to one of Claims 1 to 14.

16. Motor vehicle according to Claim 15, **characterized in that** the odorizing device is fastened at a chosen place on the dashboard, particularly in the glove box (12), within the console of the vehicle.

## Patentansprüche

1. Vorrichtung zum Beduften des Innenraums eines Kraftfahrzeugs von der Art, die mindestens eine einen aromatischen Wirkstoff enthaltende, austauschbare Patrone aufweist, die von einem zu beduftenden Luftstrom durchströmt werden kann, um einen bedufteten Luftstrom zu erzeugen,
**dadurch gekennzeichnet, dass** sie eine Platte (20), die mit einem Sitz (32) versehen ist, in den ein Eingangsfenster (34) für den zu beduftenden Luftstrom und ein Ausgangsfenster (36) für den bedufteten Luftstrom münden, eine Hülle (68) von allgemein zylindrischer Form, die von der Platte (20) getragen wird und mindestens eine einen aromatischen Wirkstoff enthaltende Patrone (78) aufnehmen kann, die in axialer Richtung angeordnet ist, wobei die Patrone einen Eingang (100) und einen Ausgang (102) hat, die in der gleichen Endfläche (96) angeordnet sind, sowie ein Einstellorgan (56) aufweist, das zwischen den Sitz (32) der Platte (20) und die Endfläche der Patrone eingefügt und in der Lage ist, wahlweise entweder in eine Lagerstellung, in der der aromatische Wirkstoff der Patrone weder mit dem Eingangsfenster (34) noch mit dem Ausgangsfenster (36) in Verbindung steht, oder in mindestens eine Diffusionsstellung gebracht zu werden, in der ein zu beduftender Luftstrom in den Eingang (100) einer ausgewählten Patrone eintreten und sich mit dem aromatischen Wirkstoff dieser Patrone beladen kann, um einen bedufteten Luftstrom zu erzeugen, der durch den Ausgang (102) der Patrone austritt.

2. Vorrichtung nach Anspruch 1, **dadurch gekennzeichnet, dass** die Platte (20) einen Eingangsstutzen (38) und einen Ausgangsstutzen (40) aufweist, die je mit dem Eingangsfenster (34) bzw. dem Ausgangsfenster (36) in Verbindung stehen.

3. Vorrichtung nach einem der Ansprüche 1, und 2, **dadurch gekennzeichnet, dass** der Sitz, (32) der Platte (20) ringförmig ist, dass das Eingangsfenster (34) und das Ausgangsfenster (36) in diametral entgegengesetzten Bereichen münden, und dass das Einstellorgan (56) eine drehbare Scheibe ist, die mindestens zwei Öffnungen (58) aufweist, die in ausgewählten Bereichen ausgebildet sind, welche in einer Ausgabestellung mit dem Eingangsfenster (34) bzw. dem Ausgangsfenster (36) in Verbindung stehen.

4. Vorrichtung nach Anspruch 3, **dadurch gekennzeichnet, dass** die Platte (20) eine zentrale Öffnung (42) für den Durchlass einer drehenden Welle (26) aufweist, die mit dem Einstellorgan (56) verbunden ist.

5. Vorrichtung nach einem der Ansprüche 1 bis 4, **dadurch gekennzeichnet, dass** sie einen Stellantrieb (24) aufweist, der von der Platte (20) getragen wird und das Einstellorgan (56) in Drehung versetzen kann, um es in eine ausgewählte Winkelstellung zu bringen.

6. Vorrichtung nach Anspruch 5, **dadurch gekennzeichnet, dass** der Stellantrieb (24) aus den mechanischen, elektrischen und pneumatischen Stellantrieben ausgewählt wird.

7. Vorrichtung nach einem der Ansprüche 1 bis 6, **dadurch gekennzeichnet, dass** die Patrone (68) mindestens eine innere Trennwand (98) aufweist, um einen U-förmigen oder zickzackförmigen Umlaufkreis für den Luftstrom zwischen dem Eingang (100) und dem Ausgang (102) der Patrone zu definieren.

8. Vorrichtung nach einem der Ansprüche 1 bis 7, **dadurch gekennzeichnet, dass** sie Mittel zur Identifizierung der Patrone aufweist.

9. Vorrichtung nach Anspruch 8, **dadurch gekennzeichnet, dass** die Identifizierungsmittel von einem Stopfen (80) der Patrone (78) getragen werden, der der Endfläche der Patrone gegenüber liegt.

10. Vorrichtung nach Anspruch 8, **dadurch gekennzeichnet, dass** die Identifizierungsmittel Erkennungsmittel von der Art Chip, elektrischer Kontakt, Strichcode sind.

11. Vorrichtung nach einem der Ansprüche 1 bis 10, **dadurch gekennzeichnet, dass** die Hülle (68) Haltemittel (86, 88) aufweist, um die Patrone in der Hülle zu blockieren.

12. Vorrichtung nach einem der Ansprüche 1 bis 11, **dadurch gekennzeichnet, dass** die Hülle (68) zwei Patronen (78) aufnehmen kann, die in axialer Richtung nebeneinander angeordnet sind.

13. Vorrichtung nach Anspruch 12, **dadurch gekennzeichnet, dass** die Hülle (68) eine zylindrische Wand (72) aufweist, die mit einer diametral verlaufenden, inneren Trennwand (74) versehen ist, die zwei Sitze (76) von allgemein halbzylindrischer Form definieren kann, um zwei Patronen von allgemein halbzylindrischer Form aufzunehmen, und dass die Hülle (68) ein offenes Ende (70) aufweist, das einen Zugang für das Einsetzen oder Entnehmen der Patronen bildet.

14. Vorrichtung nach einem der Ansprüche 1 bis 13, **dadurch gekennzeichnet, dass** sie Befestigungsmittel zur Montage an einer ausgewählten Stelle eines Kraftfahrzeugs aufweist.

15. Kraftfahrzeug, **dadurch gekennzeichnet, dass** es mit einer Beduftungsvorrichtung nach einem der Ansprüche 1 bis 14 ausgestattet ist.

16. Kraftfahrzeug nach Anspruch 15, **dadurch gekennzeichnet, dass** die Beduftungsvorrichtung an einer ausgewählten Stelle des Armaturenbretts, insbesondere im Handschuhfach (12), in Höhe der Konsole des Fahrzeugs befestigt ist.
